# EUROPEAN PATENT SPECIFICATION

(11) **EP 4 447 852 B1**
(45) Date of publication and mention of the grant of the patent: **23.04.2025**
(21) Application number: 22838706.4
(22) Date of filing: 14.12.2022
(51) Int. Cl.: A61C 17/22, A46B 15/00, A61C 19/04, A46B 5/00

(54) **A SYSTEM FOR ORAL SOFT TISSUE ANALYSIS**
SYSTEM ZUR ANALYSE VON ORALEM WEICHGEWEBE
SYSTÈME D'ANALYSE PAR VOIE ORALE DE TISSUS MOUS

(30) Priority: 16.12.2021 US 202163290090 P; 26.04.2022 EP 22170113
(43) Date of publication of application: 23.10.2024
(73) Proprietor: Koninklijke Philips N.V., 5656 AG Eindhoven (NL)
(72) Inventor: BRANDÃO SILVA, Priscilla, 5656 AG Eindhoven (NL); RMAILE, Amir Hussein, 5656 AG Eindhoven (NL); JOHNSON, Mark Thomas, 5656 AG Eindhoven (NL); GERHARDT, Lutz Christian, 5656 AG Eindhoven (NL); KOOIJMAN, Gerben, 5656AG Eindhoven (NL)
(74) Representative: Philips Intellectual Property & Standards
(86) International application number: PCT/EP2022/085761
(87) International publication number: WO 2023/110968

(56) References cited:
- WO-A1-2020/212248
- CN-A- 112 107 386
- US-B2- 9 333 060

## Description

### FIELD OF THE INVENTION

The present invention relates to analysis of the properties of oral soft tissue.

### BACKGROUND OF THE INVENTION

The analysis of oral soft tissues, such as the gums, cheeks and tongue, is an important part of a dental examination. For example, the identification of gum recession and gum inflammation is one of the elements of an examination by a dentist. Gum recession and inflammation develop slowly and if left untreated can result in serious conditions such as periodontitis. Periodontitis is a chronic gum disease which has been reported as affecting approximately half of the adult US population, yet more than half of those affected are not diagnosed as such by their dentist. The key reason is that the screening process is time consuming and painful and can only be done by a dental practitioner.

It would therefore be desirable to provide a system able to analyze soft tissue properties, for example to be able to detect gum recession and/or gum inflammation as efficiently as possible, and in particular to reduce the discomfort to a patient of a prolonged examination.

The condition of the gums can for example be assessed using impact and shaker tests. These tests are widely used in structural dynamic analysis for the determination of impulse response, mode shapes, resonances and damping of the test structure by analysis of time and, mainly, frequency responses.

In impact tests, an impact generates a half-sine-shaped force pulse of short duration. From the classical Hertzian theory, the contact force can be assumed to be of the form f = K₁.q^{α}, where K₁ is the contact stiffness, q is the relative displacement between the bodies in contact, and α is the nonlinear coefficient. The magnitude and the duration of the force pulse depends on the non-linear contact stiffness. For a given impact magnitude (i.e. momentum, velocity and inertia of impactor), soft contact surfaces generate long pulses of small force amplitude, and stiff surfaces generate short pulses of large amplitude.

The article of M. Kobusch, L. Klaus and L. Muñiz Mendoza: Investigation of impact hammer calibrations, IMEKO 23rd TC3, 13th TC5 and 4 th TC22 International Conference 30 May to 1 June, 2017, Helsinki, Finland, shows impact signals measured with tips of different material stiffness. Time domain signals (sensor signal versus time) are shown, from which different pulse height and pulse width characteristics for the time domain signals can be observed. The frequency domain response can also be analyzed. (sensitivity versus frequency).

In the frequency domain, long pulses correspond to lower frequency contents, and short pulses correspond to higher frequency contents.

It is shown that while the pulse width increases, the pulse height decreases with decreasing surface material stiffness. For instance, from pulse shape characteristics, it is possible to differentiate between rubber samples of different stiffness, as well as between stiff (steel) and soft (rubber) materials.

It is also well-known that soft biological tissues show frequency dependent mechanical properties similar to those of soft elastomers like silicone rubber

As an alternative to impact excitation, dynamic mechanical analysis (DMA) can be performed by frequency (or temperature) sweeps (for example with a range 10-1000 Hz) to derive viscoelastic properties such as storage, loss modulus and loss factor (or damping factor), which can be used to characterize materials. In DMA, a sinusoidal tapping motion ('stress') is applied and the sinusoidal response ('strain') of the sensing bristles, preferably kept in stationary contact with the target region during excitation, is recorded.

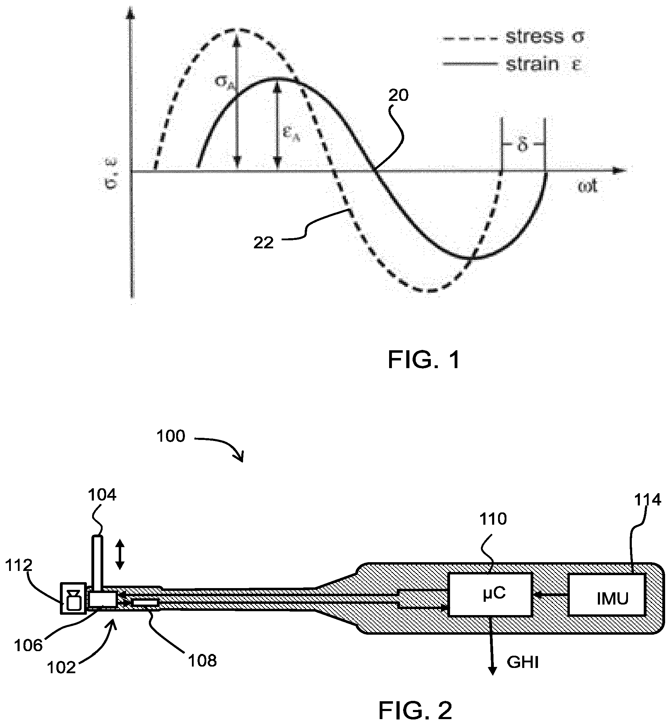

Figure 1 shows the stress-strain response of a sample subjected to sinusoidal tapping loading. Plot 20 is the strain versus time and plot 22 is the stress versus time.

It is known to incorporate sensing functionality into an oral care device, such as a toothbrush or flossing system. For example, it is known to use pressure sensing relating to a delivered fluid flow, to detect a transition from the hard tooth plaque to softer tissue. A pressure change results from the difference in flow resistance caused by the different characteristics of the different surfaces.

Such pressure sensing can for example differentiate between the teeth and gums, and also the interdental spaces. This information can then be used to control an oral irrigation or flossing system for example to use oral irrigation liquid more efficiently.

An oral treatment device such as a toothbrush or flossing head is for example known which incorporates a fluid delivery tube through which liquid e.g. air is pumped to an output nozzle. An interdental space can for example be detected based on a pressure sensor detecting a pressure drop or a flow rate increase in the fluid delivery tube. The flow of fluid from the fluid delivery tube is more obstructed when the nozzle is in contact with a tooth relative to when the aperture is away from said contact. This enables detection of more soft tissue, or lack of tissue such as an interdental space.

Detection of gum recession of inflammation however remains challenging and has not been achieved with integrated sensing approaches as discussed above. It would be of interest to have a quantitative approach for assessing changes in gum tissue properties to identify early development of gum disease, allowing for risk stratification/triaging related to gum disease. In addition, it would be beneficial if the measurements could be carried out during a user daily oral care/hygiene routine, such as during a tooth brushing session.

US 9 333 060 discloses a fluid jet dental cleaning device, in which the jet is controlled based on sensing the condition of the tissue faced by the jet, for example to clean teeth at high pressure and reduce the pressure applied to the gum. In one example the softness of gums can be detected in order to reduce the injection pressure.

WO 2020/212248 discloses an fluid jetting oral care device which seeks to identify the location of the fluid jetting head relative to an interdental space.

CN 112 107 386 discloses a system for collecting oral health data. Various sensors may be used for collecting oral health data such as gyroscopes, acceleration sensors, pressure sensors, image sensors, ultrasonic sensors, spectral test sensors, pH sensors, temperature sensors.

### SUMMARY OF THE INVENTION

The invention is defined by the claims.

According to examples in accordance with an aspect of the invention, there is provided a system for monitoring oral soft tissue properties, comprising:
an oral monitoring head comprising at least one sensing element;
an actuator for imparting a motion to the at least one sensing element;
a force or strain sensor or motion sensor for monitoring a force or strain or motion associated with the at least one sensing element and producing a force or strain or motion signal;
a controller adapted to:
   control the actuator;
   receive the force or strain or motion signal; and
   analyze the force or strain or motion signal to derive viscoelastic material properties for the material in contact with the at least one sensing element.

The invention thus provides a system for monitoring the properties of oral soft tissues, in particular the gums, by probing the soft tissue with a sensing element and analyzing a force or strain to derive a material properties. The sensing element is a tip of a probe for contacting the soft tissue surface. The force or strain sensor is then coupled to that probe.

The system is for monitoring gum health, and the controller is adapted to provide a gum health indication based on the viscoelastic material properties. The gum health indication for example may simply comprise a healthy indication or an inflamed indication, although in more advanced examples a degree of severity may be obtained. The system may also provide an indication or even measure of gum recession in some advanced examples.

The at least one sensing element for example comprises a fiber, wherein the force at the tip is measured or a strain is determined resulting from bending of the fiber.

The motion imparted may be towards and away from the soft tissue surface (so providing a tapping against the soft tissue surface with motion perpendicular to the soft tissue surface) or it may be a frequency signal applied to the proximal end of the sensing element while the distal end remains in contact with the soft tissue surface.

The controller may be further adapted to receive images of the oral cavity.

A soft tissue e.g. gum color can for example then be determined in the vicinity of the at least one sensing element. Thus, in the case of analysis of the gums, the viscoelastic material properties and the gum color may be combined to derive the gum health indication.

The system may then further comprise an imaging camera for capturing the images of the oral cavity (such as built into the oral monitoring head), although this imaging camera may be a separate device, such as a mobile phone of the user of the system.

The system may comprise a head motion or position sensor for detecting the location of the oral monitoring head.

This can be used to identify the soft tissue location to which the viscoelastic material properties relate. Image analysis may be used to identify the in-mouth location.

Image analysis may be combined with a separate location sensing solution, and then the location may be used to map the location of the oral monitoring head with the location in the captured images. The combination of image information and location information for example enables the system to determine when the oral monitoring head is located at a gum or other oral soft tissue or at a tooth, or indeed when it is moving across a tooth/gum boundary.

The head motion or position sensor for example comprises an inertia measurement unit comprising an accelerometer and gyroscope. A 6-axis measurement unit may be used so that positions and movements in 3D space can be tracked.

The controller may be adapted to analyze the force or strain signal in the time domain and the frequency domain. This enables the material properties to be determined in terms of temporal characteristics (i.e. damping effects) and frequency characteristics (i.e. resonance effects).

The controller may for example be adapted to determine a minimum gum stiffness during a monitoring period. This corresponds to a state of maximum inflammation.

The controller may be adapted to determine whether the at least one sensing element has contacted a tooth surface or a gum surface, and only when a gum surface has been contacted to determine the gum health indication. Thus, false indications can be avoided by detecting when the at least one sensing element is at a gum surface. This may be derived from the signal analysis and/or the image analysis.

For all possible types of oral soft tissue, the controller is for example adapted to control the actuator to apply a frequency sweep to the motion imparted to the at least one sensing element. Thus, the material response to different excitation frequencies is analyzed as part of the determination of the viscoelastic material properties. In this case, the part of the sensing element in contact with the soft tissue may be static, and the motion is applied to an opposite end.

The system may thus use a dynamic mechanical analysis (DMA). In such a DMA, a sinusoidal force (stress σ) is applied to the soft tissue and the resulting displacement (strain) is measured. For a perfectly elastic solid, the resulting strain and the stress will be perfectly in phase. For a purely viscous fluid, there will be a 90 degree phase lag of strain with respect to stress. Viscoelastic properties result in a phase lag during DMA testing.

The force or strain or motion sensor for example comprises one or more of:
A piezoelectric sensor;
a strain gauge;
a fiber-Bragg grating;
an accelerometer;
a Fabry-Perot sensor.

In a first set of examples, the system may be a standalone system for monitoring soft tissue properties. Thus, the sole purpose of the system may be for analysis of soft tissues.

In other examples, the soft tissue analysis, e.g. gum health analysis, is combined with an oral care device having another primary function.

For example, in a second set of examples, the invention provides a powered toothbrush device for implementing an oral care routine of tooth brushing, comprising:
a handle part;
a brushing head comprising an arrangement of bristles; and
the system defined above, wherein the at least one sensing element is part of the brushing head, with the at least one sensing element within or adjacent the arrangement of bristles.

Thus, the gum health analysis can take place as part of a tooth brushing routine.

In a third set of examples, the invention provides a powered brushing mouthpiece device for implementing an oral care routine of mouthpiece tooth cleaning, comprising:
a mouthpiece arch;
a brushing arrangement comprising an arrangement of bristles in the mouthpiece arch; and
the system defined above, wherein the at least one sensing element is part of the mouthpiece arch, with the at least one sensing element within or adjacent the arrangement of bristles.

The brushing mouthpiece device for example has a handle, which remains outside the mouth whereas the mouthpiece arch fits inside the mouth.

Thus, the gum health analysis can take place as part of a tooth cleaning routine using a brushing mouthpiece. The at least one sensing element may for example comprises a set of sensing elements, distributed around the mouthpiece arch

In a fourth set of examples, the invention provides an oral irrigator device for implementing an oral care routine of oral irrigation or flossing, comprising:
a handle part;
an oral irrigator or flossing head;
a jetting tube between the handle part and the oral irrigator or flossing head; and
the system defined above, wherein the at least one sensing element is part of the oral irrigator or flossing head.

Thus, the gum health analysis can take place as part of an oral irrigation or flossing routine.

In each of these devices, the controller may thus be adapted to determine the soft tissue properties, and optionally a gum health indication, during the oral care routine.

The invention also provides a computer-implemented method for monitoring oral soft tissue properties, comprising:
controlling an actuator to impart a motion to at least one sensing element of oral monitoring head, for driving the at least one sensing element towards and away from the soft tissue surface;
receiving a force or strain or motion signal obtained by monitoring a force or strain or motion associated with the at least one sensing element;
analyzing the force or strain signal or motion to derive viscoelastic material properties for the material in contact with the at least one sensing element.

The invention also provides a computer program comprising computer program code which is adapted, when said program is run on a controller of the system for monitoring oral soft tissue properties as defined to implement the above method.

These and other aspects of the invention will be apparent from and elucidated with reference to the embodiment(s) described hereinafter.

### BRIEF DESCRIPTION OF THE DRAWINGS

For a better understanding of the invention, and to show more clearly how it may be carried into effect, reference will now be made, by way of example only, to the accompanying drawings, in which:
Figure 1 shows the stress-strain response of a sample subjected to sinusoidal tapping loading;
Figure 2 shows a system for monitoring soft tissue properties, for example for assessing gum health;
Figure 3 shows schematically the head section of a toothbrush which includes bristles as well as a gum health sensing element;
Figure 4 shows schematically a dental cleaning mouthpiece which includes cleaning bristles as well as a gum health sensing element;
Figure 5 shows an arrangement of three sensing elements at different heights up the tooth and gum surface;
Figure 6 shows different sensing elements at different angles;
Figure 7 shows a measure of differential stiffness over time as gum recessing develops;
Figure 8 shows a device with an oral irrigator function combined with a toothbrush head; and
Figure 9 shows an arrangement which can perform both an air flossing function or a liquid oral irrigation function.

### DETAILED DESCRIPTION OF THE EMBODIMENTS

The invention will be described with reference to the Figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

The invention provides a system for monitoring properties of oral soft tissues. An oral monitoring head comprises at least one sensing element such as a probe which can be provided among cleaning bristles. A tapping motion is imparted to the at least one sensing element and the force or strain or motion associated with the at least one sensing element (such as a reaction force from the soft tissue tissue) is monitored to produce a force or strain or motion signal. From this signal, viscoelastic material properties for the material in contact with the at least one sensing element are derived. In one example, a gum health indication can be provided.

The invention may be implemented as a device only for oral soft tissue analysis or as part of a toothbrush, or a cleaning mouthpiece, or an oral irrigator or a flossing system, or part of a system combining two or more of tooth brushing, oral irrigation (e.g. using a liquid), flossing (e.g. using air) and cleaning using a mouthpiece.

The invention will be described with reference to a system for monitoring gum health. However, more generally the system can be used to analyze the material properties of oral soft tissue more generally, such as the gums, tongue or cheeks.

Figure 2 shows system 100 specifically for monitoring gum health. The system 100 comprises an oral monitoring head 102 comprising at least one sensing element 104 in the form of an elongate fiber or probe. An actuator 106 (e.g. drive train, motor, and mechanical transmission unit in the handle or head of the system) is provided for imparting a motion to the at least one sensing element 104 against the tissue being analyzed. The motion is along an elongate axis of the sensing element. The actuator may be in the brush head as shown, but equally it may be implemented by the motor and drive train of the toothbrush, normally found in the handle of the toothbrush.

A sensor 108 monitors a reaction from the soft tissue. In a preferred set of example, the sensor is a force or strain sensor for monitoring the force or strain associated with the at least one sensing element 104, and it produces a force or strain signal.

However, the reaction may be assessed using a motion sensor. For example an accelerometer (and optionally also a gyroscope) may be used to monitor the motion of the tip of the sensor, so that displacement, velocity and acceleration of the sensor can be monitored. This motion will be influenced by the nature of the soft tissue, i.e. the damping it provides to the motion of the sensor tip caused by a particular tip actuation profile. Thus, the sensor is generally a force or strain or motion sensor.

Typically, the sensing function and the actuation function are decoupled, but this is not essential . For example, a self-sensing actuator could be formed for example by using piezoelectric materials

The signals of interest are those which are generated while the sensing element 104 remains in contact with the gum tissue, since once it is retracted so that contact is lost, there is no more information to be gathered.

A controller 110 generates the drive signal to the actuator 106 and also receives the force or strain signal from the sensor 108. The controller 110 analyzes at least the force or strain signal to derive mechanical or viscoelastic material properties, such as tissue stiffness, for the material in contact with the sensing element 104. Based on these viscoelastic material properties, a gum health indication GHI is provided.

The stiffness measurement can for example be synchronized with the sensing element motion, for example to measure the stiffness at a maximum movement depth.

The invention thus provides a system for monitoring gum health by probing the gum with a sensing element 104 and analyzing a force or strain or motion to derive a health indication. The gum health indication for example may simply comprise a healthy indication or an inflamed indication, although in more advanced examples described below a degree of gum health severity may be obtained. The system may also provide an indication or even measure of gum recession in some further examples.

Figure 2 shows a refinement in which a camera 112 is also provided for capturing images of the oral cavity. By way of example, the images may be used to identify a gum color for the gum region being probed by the sensing element 104. An integrated camera for example uses micro-CMOS technology. The camera 112 may of course be part of a separate device, such as a home-use intra-oral scanner, or a smartphone camera.

Figure 2 also shows a refinement in which an inertia monitoring unit 114 is also provided. This can be used to identify the gum location to which the gum health indication relates. The inertia monitoring unit 114 functions as a motion or position sensor for detecting the location of the oral monitoring head.

Location information may also be used to provide registration between a captured camera image and the location of the oral monitoring head. The combination of image information and location information for example enables the system to determine when the oral monitoring head is located at a gum or at a tooth, or indeed when it is moving across a tooth/gum boundary.

The inertia monitoring unit comprises an accelerometer and gyroscope. A 6-axis measurement unit may be used so that positions and movements in 3D space can be tracked.

The force or strain sensing capability for example makes use of a piezoelectric sensor, a strain gauge, fiber Bragg grating or a Fabry-Perot sensor, in order to acquire the force or strain pulse shape during contact between the tip of the sensing element 104 and the target region (i.e. gums or teeth), or to acquire the sensing element deformation characteristics in response to a sinusoidal excitation.

Suitable driving schemes can include an impulse drive signal, a frequency sweep towards an increasing frequency or towards a decreasing frequency, or cyclic up-down or down-up frequency sweeps. This is enabled by the controller when actuating the sensing element. A frequency sweep is for example in a frequency range of 10-1000 Hz, and during the sweep the sensor signal response is fed back to the controller.

The relationship between the actuator signal and the sensor signal enables the viscoelastic material properties to be determined.

For an impulse test, an impulsive motion of theoretically infinitely small duration is applied to the probe against the gum, which causes an almost flat response (i.e. constant amplitude) in the frequency domain. The force measurement can be either at the tip of the sensing element or in the head (i.e. at the proximal end of the sensing probe). This impulse test is thus analogous to an impact hammer modal testing.

The frequency sweep test is analogous to shaker modal testing. In this case, a periodic oscillatory or random signal excitation can be applied.

In both the frequency sweep case and the the impulse case, the probe is moved in and out to create a tapping motion in order to keep the tip as much as possible in contact and almost stationary with respect to the target surface (gums). In both cases, time and frequency domain responses provide an indication of the resonance frequencies and damping levels, which will allow the identification of stiffness and viscoelastic characteristics of the target surface.

The sensing element for example has the structure of a bristle, and indeed it may perform a cleaning function in the same way as other non-sensing bristles of a toothbrush head (as discussed further below). The sensing bristles for example comprise a piezoelectric polymer fiber or a fiber Bragg grating, or an optical fiber based Fabry-Perot interferometer sensor.

The actuator may be at the oral monitoring head, or it may be in a handle part of the device, with a drivetrain between the handle part and the oral monitoring head.

After actuating the sensing element, for example in a tapping mode, with a controlled mechanical or motion stimulus of the sensing element, the analysis of the response of the sensor signal in the time and frequency domain can first determine whether a tooth or gum surface has been hit. These target regions have quite distinguishable stiffness and damping properties. Secondly, if the gum surface has been hit, the gum condition (healthy or inflamed) can then be is assessed.

Variations on the gum condition can be detected by differential changes in stiffness (or pulse shape time/frequency domain characteristics), color (chromaticity coordinates from gum image analysis), and even texture from image analysis.

An assessment that the gum is healthy may for example be made by deriving a health ratio H = r /V relating to gum health, wherein a vibration signal characteristic parameter is represented as V (e.g. a 1^{st} harmonic frequency, amplitude or pulse width) and a chromaticity coordinate is represented as r. This ratio H can then be compared to a threshold value, below which the gums are considered healthy, and above which, they are assessed as inflamed.

The chromaticity coordinate represents the red content of a color (where for blue or green r=0, for red r=1).

The nature of the gum health indication will depend on the accuracy of location sensing available.

For example, with relatively coarse location sensing, a minimum gum stiffness measured in a session over time may be tracked, and the ratio H is evaluated and compared to a specified threshold for concluding a general healthy gum condition. Coarse location sensing may be used for example to make sure that in a brushing session the mouth is sufficiently covered, such that the obtained stiffness measurements are fair to compare to a previous brushing session.

With more accurate location sensing, the contact location can be determined based on the position of the sensing element. By scanning the oral cavity using the system, the gum location can be mapped into a 3D model, and tracked over time. This then enables a localized assessment of gum health, and even an assessment of gum recession based on the location of the gum/tooth boundaries.

In operation of the system of Figure 2, the user (for example an oral health care professional) holds the system to the teeth or gum surface. The sensing element is moved across the tooth/gum boundary. Under tapping excitation, periodic impacts of the sensing element against the oral surface are promoted while moving the sensing element, in order to implement impact-based analysis. Alternatively (or additionally), the sensing element is kept with one end stationary and in contact with the target surface and subjected to sinusoidal excitation at the opposite end, to implement dynamic mechanical analysis (DMA). These analysis modes are described above.

When an inertia monitoring unit is used, the sensing element output and the location information derived from the IMU are synchronized and recorded. The signals can be analyzed either in the controller for a stand-alone system probe or the data can be transferred to be processed in a connected smartphone or computer or even remotely in the cloud.

The example above shows a system specifically for gum health analysis. The system may however be combined with an oral care device which has the main purpose of performing an oral care routine.

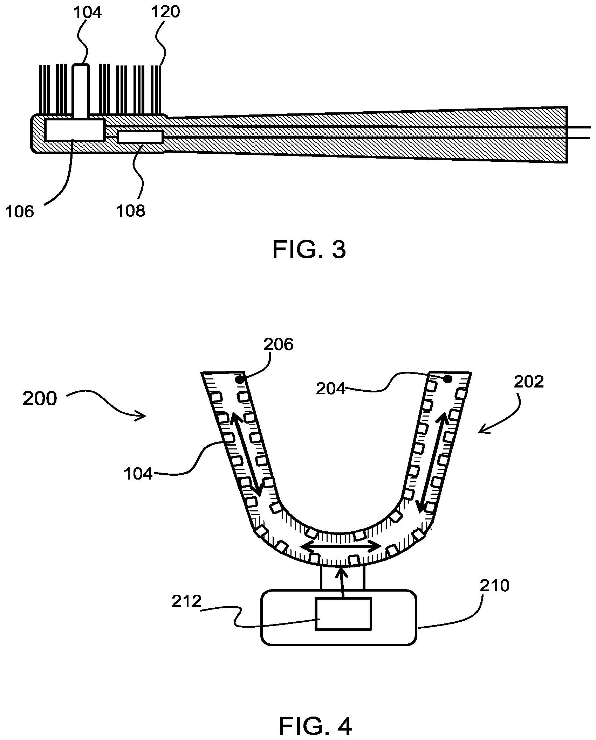

Figure 3 shows schematically the head section of a toothbrush which includes bristles 120 as well as the sensing element 104 of the system described above. The sensing element 104 is thus incorporated into the toothbrush head. The tip of the sensing element is positioned at a suitable position between or close to the bristle tufts 120 of the toothbrush head. The sensing element 104 protrudes from the head section in the same (or largely the same) direction as the bristles 120 of the toothbrush and indeed the sensing element may itself perform the same cleaning function as the non-sensing bristles.

In operation, the sensing element signal is monitored in the same way as explained above and the gum assessment is defined by similar signal traces.

The gum assessment may occur during a regular oral cleaning session (whether using an impulse test or a short duration frequency sweep test). In the case of (power) toothbrushes, one or more, or even all of the bristles, may be sensing bristles with the dual function of brushing and sensing.

The traces are captured as the user moves the toothbrush head around the mouth. When the optional location sensing described above is provided, and the system is also for mapping the gum and tooth location, the location sensing may be used to identify when the user moves the toothbrush head across the tooth/gum boundary. The location sensing may for example be used to identify specifically when a vertical movement is made and the probe has most likely crossed the tooth/gum boundary. The gum and tooth positions may then be assessed as part of a mapping function.

For improved performance, guidance on the probe usage (e.g. device positioning and path to be followed) can be provided via an app, and a dedicated mode can be incorporated into the device for periodic gum scanning and assessment to be performed before or after a standard brushing session.

Figure 4 shows schematically the dental cleaning mouthpiece which incorporates the multiple sensing elements 104 (for different gum locations) of the system described above.

Figure 4 shows one channel a mouthpiece-based dental cleaning system. The system 200 comprises a mouthpiece 202 for insertion in the mouth of the user and an external part 210 for positioning outside the mouth of the user, in front of the user's mouth. The external part 210 for example functions as a handle of the system.

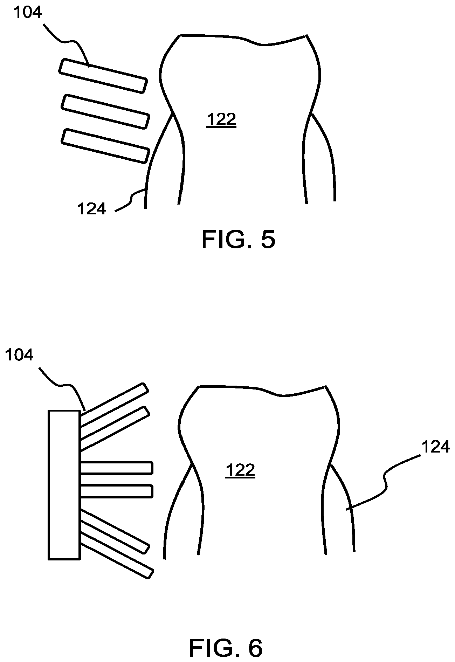

The mouthpiece comprises a first U-shaped (or equivalently C-shaped) channel 204 for receiving the teeth of one jaw, and a second U-shaped channel for receiving the teeth of the other jaw. Each channel defines a mouthpiece arch. Figure 5 is for example looking down on the first channel 204 which open to the top, and the second channel is underneath, open to the bottom.

The mouthpiece may instead have a J-shape for fitting generally to one side of the jaw. In cross section, each channel may have a U-shape to define a channel opening for receiving the teeth. However, the two channels may be combined into one structure to create a H-shape cross section, with top and bottom channel openings.

The overall mouthpiece is bitten onto by the user with their teeth in the two channels. The system may instead have only one channel, in which case the cleaning may be performed one jaw at a time.

The channel 204 comprises a cleaning arrangement 206 such as bristles that face the teeth of the user within the mouthpiece channel. The bristles are for example driven parallel to the tooth surfaces as shown by the arrows.

The external part 210 has an actuator 212 for applying movement to an inner arch and/or an outer arch of the channel shape (relative to a fixed base). For a system with two U-shaped channels, a shared actuator may be used.

The sensing elements are part of each mouthpiece arch, with the at least one sensing element 104 within or adjacent the bristles of the cleaning arrangement.

In another example (not shown in the Figures) the gum health analysis can be incorporated into a cleaning mouthpiece. Multiple sensing elements are then distributed at suitable locations within a mouthpiece arch (because the mouthpiece is not moved around the oral cavity) and the sensing elements are within or adjacent the arrangement of bristles. Limited or no location sensing is needed, because of the limited motion around the oral cavity.

Some or all of the mouthpiece bristles may again have a dual function of brushing and sensing. As in the previous example, a dedicated mode for periodic gum health scanning and assessment before or after a mouthpiece cleaning session can be incorporated into the oral care device.

There may be different configurations of sensing element, for example sensing elements at different positions and optionally also different orientations relative to the tooth or gum. The orientation and position of one sensing element may be arranged to measure gum tissue (e.g. positioned closed to the gum line), while another orientation may be for measuring the tooth surface.

Figure 5 shows three sensing elements 104 at different heights up the tooth and gum surface with the mouthpiece arch. By tracking the differentially measured stiffness over time between different sensing elements, gum recession can be detected, based on sensing elements transitioning from detection of gum tissue to detection of a tooth surface. The, if gum recession develops, one of the gum sensing elements will start to measure a stiffness corresponding to a tooth, so that a differential stiffness measure decreases. If a critical threshold is reached, the user can be advised to see a dentist, or an automatic triaging alert is sent to the dentist for appointment scheduling.

Figure 6 shows different sensing element at different angles, so that some are directed to the tooth surface, some are directed to the gum/tooth boundary and some are directed to the gums.

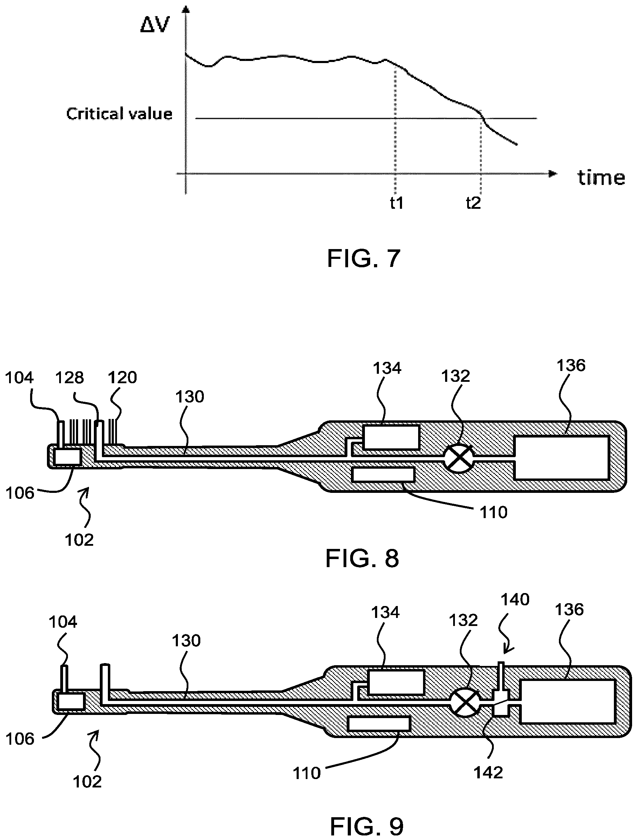

Figure 7 shows a measure of differential stiffness over time, namely the difference in stiffness between a sensing element positioned for tooth detection and a sensing element positioned for gum detection. The difference may instead be a ratio.

At a first point in time t1 gum recessing starts to develop. At a second point in time t2 an alert is triggered.

The gum health analysis may also be applied to an oral flossing and/or irrigation system.

Figure 8 shows a device with an oral irrigator function combined with a toothbrush head 102.

The toothbrush head 102 has the sensing element 104 and actuator 106 as described above (the other parts are not shown to keep the diagram simple).

The system additionally comprises a nozzle 128 which is also applied to the teeth and gums when the head 102 is used. The nozzle 128 is the distal end of a fluid delivery tube 130. The proximal end is configured to receive a fluid from a reservoir 136 or it may use ambient air as a delivery fluid.

A pump 132 is configured to pump the fluid through the fluid delivery tube 130 to the nozzle 128. In this example, the fluid reservoir 136, pump 132 and sensor 134 are part of the toothbrush. However, these components may be separate to the toothbrush and connected by the fluid delivery tube 130.

In this example, the oral irrigator function of the combined toothbrush and oral irrigator includes its own sensing functionality (in addition to the gum health sensing), and a sensor 134 is provided for sensing a pressure in the fluid delivery tube 130 or a flow rate along the fluid delivery tube 130. In one example, the sensor 134 is a pressure sensor. However, the sensor 134 could also be a flow sensor or a combined sensor which can measure flow rate and pressure inside the fluid delivery tube.

The sensing function provides an alternative or additional way to detect when the head is against a tooth or gum surface. In particular, the fluid resistance at the nozzle 128 will influence the flow rate and pressure. Soft gum tissue for example closes the nozzle more effectively than hard tooth tissue, so that a higher pressure and lower flow rate is observed when contact is made with the gums. An even lower pressure and higher flow rate is observed when there is no contact, e.g. when the nozzle is aligned with an interdental space. This approach is known, for example for controlling the delivery of oral irrigation liquid in dependence on the position of the head, i.e. depending on whether it is aligned with the teeth or aligned with an interdental space.

Figure 8 shows the delivery of a liquid from a reservoir 136, and such liquid delivery is known for an oral irrigator. Air delivery is for example also known for an air flossing system.

Figure 9 shows an arrangement which can deliver air or liquid to the delivery tube 130, so that it can perform both an air flossing function or a liquid oral irrigation function. In this example, the system is no longer also for tooth brushing.

The pump 132 is able to provide pumping of air by means of an air inlet 140 and a switch 142 between the fluid reservoir 136 and the air inlet 140. The switch 142 is configured to select whether the pump 132 delivers ambient air, or a liquid from the reservoir, through the fluid delivery tube 130. Alternatively, a second fluid reservoir with air (or any gas) may be used instead of the air inlet 140. One or more air inlets 140 may also be used, to ensure that if any of the air inlets is blocked by the hand of the user, the pump can still pump air.

In this design, the gum health analysis is integrated into an oral irrigation or flossing head.

For the examples above with the health analysis integrated into an oral care device with another primary function, the gum health analysis can take place during the regular irrigation, brushing or air flossing sessions.

In examples having motion sensing and/or image-based location sensing, the device may be used to derive a 3D profile of the teeth and gums. Based on a daily oral care routine using a device with the gum health analysis, the device can be used to detect where the gums are and where the teeth are. Based on repeated analysis over time, the profile can be iterated and improved, to obtain 3D scan-like file of the teeth and gums.

To improve the 3D profile, a guided brushing (or other oral care routine) technique may be used, whereby the user is asked to follow a certain pattern when brushing, to facilitate localization, as well as to enable estimation of the vertical dimension of the teeth. For example, by asking the user to brush from the gum upwards, three different sets of spectra will be recorded, for the gums, teeth, and free space, thereby indicating the edge or height of the tooth. The user may then be instructed to move to the next tooth and so on.

The captured sensor data may be combined with a 2D photo (e.g. by superimposing), so that any missing information from one modality (e.g. camera) is provided by the other modality (e.g. sensing).

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality.

The mere fact that certain measures are recited in mutually different dependent claims does not indicate that a combination of these measures cannot be used to advantage.

The skilled person would be readily capable of developing a controller for carrying out any herein described method. The controller can be implemented in numerous ways, with software and/or hardware, to perform the various functions required. A processor is one example of a controller which employs one or more microprocessors that may be programmed using software (e.g., microcode) to perform the required functions. A controller may however be implemented with or without employing a processor, and also may be implemented as a combination of dedicated hardware to perform some functions and a processor (e.g., one or more programmed microprocessors and associated circuitry) to perform other functions.

Examples of controller components that may be employed in various embodiments of the present disclosure include, but are not limited to, conventional microprocessors, application specific integrated circuits (ASICs), and field-programmable gate arrays (FPGAs).

In various implementations, a processor or controller may be associated with one or more storage media such as volatile and non-volatile computer memory such as RAM, PROM, EPROM, and EEPROM. The storage media may be encoded with one or more programs that, when executed on one or more processors and/or controllers, perform the required functions. Various storage media may be fixed within a processor or controller or may be transportable, such that the one or more programs stored thereon can be loaded into a processor or controller.

A computer program may be stored/distributed on a suitable medium, such as an optical storage medium or a solid-state medium supplied together with or as part of other hardware, but may also be distributed in other forms, such as via the Internet or other wired or wireless telecommunication systems.

If the term "adapted to" is used in the claims or description, it is noted the term "adapted to" is intended to be equivalent to the term "configured to".

Any reference signs in the claims should not be construed as limiting the scope.

## Claims

1. A system (100) for monitoring oral soft tissue properties for monitoring gum health, comprising:
an oral monitoring head (102) comprising at least one sensing element (104);
an actuator (106) for imparting a motion to the at least one sensing element against the gum;
a force or strain or motion sensor (108) for monitoring a force or strain or motion associated with the at least one sensing element and producing a force or strain or motion signal;
a controller (110) adapted to:
control the actuator;
receive the force or strain or motion signal;
analyze the force or strain or motion signal to derive viscoelastic material properties for the gum in contact with the at least one sensing element; and
provide a gum health indication based on the viscoelastic material properties.

2. The system of claim 1, wherein the controller is further adapted to receive images of the oral cavity.

3. The system of claim 2, further comprising an imaging camera (112) for capturing the images of the oral cavity.

4. The system of any one of claims 1 to 3, comprising a head motion or position sensor (114) for detecting the location of the oral monitoring head, for example wherein the head motion or position sensor (114) comprises an inertia measurement unit comprising an accelerometer and gyroscope.

5. The system of any one of claims 1 to 4, wherein the controller (110) is adapted to analyze the force or strain or motion signal in the time domain and the frequency domain.

6. The system of any one of claims 1 to 5, wherein the controller (110) is adapted to determine a minimum gum stiffness during a monitoring period.

7. The system of any one of claims 1 to 6, wherein the controller (110) is adapted to determine whether the at least one sensing element has contacted a tooth surface or a gum surface, and only when a gum surface has been contacted to determine the gum health indication.

8. The system of any one of claims 1 to 6, wherein the controller (110) is adapted to control the actuator (106) to apply a frequency sweep to the motion imparted to the at least one sensing element.

9. The system of any one of claims 1 to 8, wherein the force or strain or motion sensor (108) comprises one or more of:
a piezoelectric sensor;
a strain gauge;
a fiber-Bragg grating;
an accelerometer;
a Fabry-Perot sensor.

10. A powered toothbrush device for implementing an oral care routine of tooth brushing, comprising:
a handle part;
a brushing head comprising an arrangement of bristles; and
the system of any one of claims 1 to 9, wherein the at least one sensing element is part of the brushing head, with the at least one sensing element within or adjacent the arrangement of bristles.

11. A powered brushing mouthpiece device for implementing an oral care routine of mouthpiece tooth cleaning, comprising:
a mouthpiece arch (204);
a brushing arrangement comprising an arrangement (206) of bristles in the mouthpiece arch; and
the system of any one of claims 1 to 9, wherein the at least one sensing element (104) is part of the mouthpiece arch, with the at least one sensing element within or adjacent the arrangement of bristles.

12. An oral irrigator device for implementing an oral care routine of oral irrigation or flossing, comprising:
a handle part;
an oral irrigator or flossing head;
a jetting tube between the handle part and the oral irrigator or flossing head; and
the system of any one of claims 1 to 9, wherein the at least one sensing element is part of the oral irrigator or flossing head

13. A computer program comprising computer program code which is adapted, when said program is run on a controller of the system for monitoring oral soft tissue properties of any one of claims 1 to 9 to implement a method comprising:
controlling an actuator to impart a motion to at least one sensing element of oral monitoring head, for driving the at least one sensing element towards and away from the gum surface;
receiving a force or strain or motion signal obtained by monitoring a force or strain or motion associated with the at least one sensing element;
analyzing the force or strain or motion signal to derive viscoelastic material properties for the gum in contact with the at least one sensing element; and
providing a gum health indication based on the viscoelastic material properties.

## Patentansprüche

1. System (100) zum Überwachen von Mundweichgewebeeigenschaften zum Überwachen von Zahnfleischgesundheit, umfassend:
einen Mundüberwachungskopf (102), der mindestens ein Sensorelement (104) umfasst;
eine Betätigungsvorrichtung (106) zum Übertragen einer Bewegung gegen das Zahnfleisch auf das mindestens eine Sensorelement;
einen Kraft- oder Dehnungs- oder Bewegungssensor (108) zum Überwachen einer mit dem mindestens einen Sensorelement verbundenen Kraft oder Dehnung oder Bewegung und zum Erzeugen eines Kraft- oder Dehnungs- oder Bewegungssignals;
eine Steuereinheit (110), die angepasst ist, um:
die Betätigungsvorrichtung zu steuern;
das Kraft- oder Dehnungs- oder Bewegungssignal zu empfangen;
das Kraft- oder Dehnungs- oder Bewegungssignal zu analysieren, um viskoelastische Materialeigenschaften für das Zahnfleisch, das mit dem mindestens einen Sensorelement in Kontakt steht, abzuleiten; und
eine Zahnfleischgesundheitsanzeige basierend auf den viskoelastischen Materialeigenschaften bereitzustellen.

2. System nach Anspruch 1, wobei die Steuereinheit weiter angepasst ist, um Bilder der Mundhöhle zu empfangen.

3. System nach Anspruch 2, weiter umfassend eine Bildgebungskamera (112) zum Aufnehmen der Bilder der Mundhöhle.

4. System nach einem der Ansprüche 1 bis 3, umfassend einen Kopfbewegungs- oder Positionssensor (114) zum Erfassen des Ortes des Mundüberwachungskopfes, wobei der Kopfbewegungs- oder Positionssensor (114) beispielsweise eine Trägheitsmesseinheit umfasst, die einen Beschleunigungsmesser und ein Gyroskop umfasst.

5. System nach einem der Ansprüche 1 bis 4, wobei die Steuereinheit (110) angepasst ist, um das Kraft- oder Dehnungs- oder Bewegungssignal im Zeitbereich und im Frequenzbereich zu analysieren.

6. System nach einem der Ansprüche 1 bis 5, wobei die Steuereinheit (110) angepasst ist, um während eines Überwachungszeitraums eine minimale Zahnfleischsteifigkeit zu bestimmen.

7. System nach einem der Ansprüche 1 bis 6, wobei die Steuereinheit (110) angepasst ist, um zu bestimmen, ob das mindestens eine Sensorelement eine Zahnoberfläche oder eine Zahnfleischoberfläche kontaktiert hat, und um, nur wenn eine Zahnfleischoberfläche kontaktiert wurde, die Zahnfleischgesundheitsanzeige zu bestimmen.

8. System nach einem der Ansprüche 1 bis 6, wobei die Steuereinheit (110) angepasst ist, um die Betätigungsvorrichtung (106) zu steuern, um einen Frequenzdurchlauf auf die Bewegung anzuwenden, die auf das mindestens eine Sensorelement übertragen wird.

9. System nach einem der Ansprüche 1 bis 8, wobei der Kraft- oder Dehnungs- oder Bewegungssensor (108) eines oder mehrere umfasst von:
einem piezoelektrischen Sensor;
einem Dehnungsmessstreifen;
einem Faser-Bragg-Gitter;
einem Beschleunigungsmesser;
einem Fabry-Perot-Sensor.

10. Elektrische Zahnbürstenvorrichtung zum Implementieren einer Mundpflegeroutine des Zähneputzens, umfassend:
einen Griffteil;
einen Bürstenkopf, der eine Anordnung von Borsten umfasst; und
das System nach einem der Ansprüche 1 bis 9, wobei das mindestens eine Sensorelement Teil des Bürstenkopfes ist, wobei sich das mindestens eine Sensorelement innerhalb oder benachbart zu der Anordnung von Borsten befindet.

11. Elektrische Bürstenmundstückvorrichtung zum Implementieren einer Mundpflegeroutine der Mundstückzahnreinigung, umfassend:
einen Mundstückbogen (204);
eine Bürstenanordnung, die eine Anordnung (206) von Borsten im Mundstückbogen umfasst; und
das System nach einem der Ansprüche 1 bis 9, wobei das mindestens eine Sensorelement (104) Teil des Mundstückbogens ist, wobei sich das mindestens eine Sensorelement innerhalb oder benachbart zu der Anordnung von Borsten befindet.

12. Mundduschenvorrichtung zum Implementieren einer Mundpflegeroutine des Mundduschens oder der Verwendung von Zahnseide, umfassend:
einen Griffteil;
ein Mundduschen- oder Zahnseidekopf;
ein Spülrohr zwischen dem Griffteil und dem Mundduschen- oder Zahnseidekopf; und
das System nach einem der Ansprüche 1 bis 9, wobei das mindestens eine Sensorelement Teil des Mundduschen- oder Zahnseidekopfes ist

13. Computerprogramm, umfassend Computerprogrammcode, der angepasst ist, um, wenn das Programm auf einer Steuereinheit des Systems zum Überwachen von Mundweichgewebeeigenschaften nach einem der Ansprüche 1 bis 9 ausgeführt wird, ein Verfahren zu implementieren, umfassend:
Steuern einer Betätigungsvorrichtung, um auf mindestens ein Sensorelement des Mundüberwachungskopfes eine Bewegung zu übertragen, um das mindestens eine Sensorelement in Richtung zu der Zahnfleischoberfläche zu treiben und von dieser weg zu treiben;
Empfangen eines Kraft- oder Dehnungs- oder Bewegungssignals, das durch Überwachen einer mit dem mindestens einen Sensorelement verbundenen Kraft oder Dehnung oder Bewegung erhalten wird;
Analysieren des Kraft- oder Dehnungs- oder Bewegungssignals, um viskoelastische Materialeigenschaften für das Zahnfleisch, das mit dem mindestens einen Sensorelement in Kontakt steht, abzuleiten; und
Bereitstellen einer Zahnfleischgesundheitsanzeige basierend auf den viskoelastischen Materialeigenschaften.

## Revendications

1. Système (100) de surveillance de propriétés de tissus mous buccaux pour surveiller la santé de la gencive, comprenant :
une tête de surveillance buccale (102) comprenant au moins un élément de détection (104) ;
un actionneur (106) pour conférer un mouvement au au moins un élément de détection contre la gencive ;
un capteur de force ou de contrainte ou de mouvement (108) pour surveiller une force ou une contrainte ou un mouvement associé au au moins un élément de détection et produire un signal de force ou de contrainte ou de mouvement ;
un dispositif de commande (110) adapté pour :
commander l'actionneur ;
recevoir le signal de force ou de contrainte ou de mouvement ;
analyser le signal de force ou de contrainte ou de mouvement pour déduire des propriétés de matériau viscoélastique pour la gencive en contact avec le au moins un élément de détection ; et
fournir une indication de santé de la gencive sur la base des propriétés de matériau viscoélastique.

2. Système selon la revendication 1, dans lequel le dispositif de commande est en outre adapté pour recevoir des images de la cavité buccale.

3. Système selon la revendication 2, comprenant en outre une caméra d'imagerie (112) pour capturer les images de la cavité buccale.

4. Système selon l'une quelconque des revendications 1 à 3, comprenant un capteur de mouvement ou de position de tête (114) pour détecter l'emplacement de la tête de surveillance buccale, par exemple dans lequel le capteur de mouvement ou de position de tête (114) comprend une unité de mesure d'inertie comprenant un accéléromètre et un gyroscope.

5. Système selon l'une quelconque des revendications 1 à 4, dans lequel le dispositif de commande (110) est adapté pour analyser le signal de force ou de contrainte ou de mouvement dans le domaine temporel et le domaine fréquentiel.

6. Système selon l'une quelconque des revendications 1 à 5, dans lequel le dispositif de commande (110) est adapté pour déterminer une rigidité de gencive minimale pendant une période de surveillance.

7. Système selon l'une quelconque des revendications 1 à 6, dans lequel le dispositif de commande (110) est adapté pour déterminer si le au moins un élément de détection est entré en contact avec une surface de dent ou une surface de gencive, et uniquement lorsqu'une surface de gencive est entrée en contact pour déterminer l'indication de santé de la gencive.

8. Système selon l'une quelconque des revendications 1 à 6, dans lequel le dispositif de commande (110) est adapté pour commander l'actionneur (106) pour appliquer un balayage de fréquence au mouvement conféré au au moins un élément de détection.

9. Système selon l'une quelconque des revendications 1 à 8, dans lequel le capteur de force ou de contrainte ou de mouvement (108) comprend un ou plusieurs parmi :
un capteur piézoélectrique ;
une jauge de contrainte ;
un réseau de Bragg à fibre ;
un accéléromètre ;
un capteur Fabry-Perot.

10. Dispositif de brosse à dents électrique pour mettre en œuvre une routine de soins buccaux de brossage de dents, comprenant :
une partie manche ;
une tête de brossage comprenant un agencement de poils ; et
le système selon l'une quelconque des revendications 1 à 9, dans lequel le au moins un élément de détection fait partie de la tête de brossage, le au moins un élément de détection se trouvant au sein de l'agencement de poils ou étant adjacent à ce dernier.

11. Dispositif d'embout buccal de brossage électrique pour mettre en œuvre une routine de soins buccaux de nettoyage dentaire d'embout buccal, comprenant :
un arc d'embout buccal (204) ;
un agencement de brossage comprenant un agencement (206) de poils dans l'arc d'embout buccal ; et
le système selon l'une quelconque des revendications 1 à 9, dans lequel le au moins un élément de détection (104) fait partie de l'arc d'embout buccal, le au moins un élément de détection se trouvant au sein de l'agencement de poils ou étant adjacent à ce dernier.

12. Dispositif d'irrigation buccale pour mettre en œuvre une routine de soins buccaux d'irrigation buccale ou de passage de fil dentaire, comprenant :
une partie manche ;
un irrigateur buccal ou une tête de fil dentaire ;
un tube de jet entre la partie manche et l'irrigateur buccal ou la tête de fil dentaire ; et
le système selon l'une quelconque des revendications 1 à 9, dans lequel le au moins un élément de détection fait partie de l'irrigateur buccal ou de la tête de fil dentaire.

13. Programme informatique comprenant du code de programme informatique qui est adapté, lorsque ledit programme est exécuté sur un dispositif de commande du système de surveillance de propriétés de tissus mous buccaux selon l'une quelconque des revendications 1 à 9, pour mettre en œuvre un procédé comprenant :
la commande d'un actionneur pour conférer un mouvement à au moins un élément de détection de la tête de surveillance buccale, pour entraîner le au moins un élément de détection vers et à l'écart de la surface de gencive ;
la réception d'un signal de force ou de contrainte ou de mouvement obtenu en surveillant une force ou une contrainte ou un mouvement associé au au moins un élément de détection ;
l'analyse du signal de force ou de contrainte ou de mouvement pour déduire des propriétés de matériau viscoélastique pour la gencive en contact avec le au moins un élément de détection ; et
la fourniture d'une indication de santé de la gencive sur la base des propriétés de matériau viscoélastique.
